# EUROPÄISCHE PATENTANMELDUNG

(11) **EP 3 895 672 A1**
(43) Veröffentlichungstag der Anmeldung: **20.10.2021**
(21) Anmeldenummer: 21158157.4
(22) Anmeldetag: 19.02.2021
(51) Int. Cl.: A61F 9/02

(54) **ELEKTROSTATISCHE EINWEGMASKE ZUM SCHUTZ DER AUGEN VOR BIOLOGISCH GEFÄHRLICHEN MIKROPARTIKELN**

(30) Priorität: 14.04.2020 DE 202020001532 U
(71) Anmelder: Kacugin, Leo, 79331 Teningen (DE)
(72) Erfinder: Kacugin, Leo, 79331 Teningen (DE)

(57) **Zusammenfassung**

Die vorliegende Erfindung betrifft eine Vorrichtung zum Schutz der Augen vor biologisch gefährlichen Mikropartikeln aus transparentem, flexiblem, elektrostatisch aktivierbarem Film, der nach dem Reiben mit den Händen das Gesicht bedeckt und ohne Befestigungselemente an der Haut haftet.

## Beschreibung

Die vorliegende Erfindung betrifft das Gebiet der Medizin und Hygiene, sie betrifft eine Vorrichtung zum Schutz bestimmter Teile der Körperoberfläche, insbesondere Augen und der umgebenden Schleimhäute, vor dem Eindringen von Aerosolen, Staub und biologisch gefährlichen Mikropartikeln.

Der Erfindung liegt die Aufgabe zu Grunde, einen einfachen Einweg-Augenschutz bereitzustellen, der einerseits einen hohen Schutz vor gefährlichen Partikel liefert und andererseits eine schnelle Zugänglichkeit bietet. Darüber hinaus sollte die Vorrichtung einfach und kostengünstig herzustellen sein.

Zurzeit existiert eine Vielzahl an Vorrichtungen zum Schutz der Augen vor Schädigung durch mechanische, chemische und biologische Einflüsse. Dabei handelt es sich um Schutzbrillen, Schutzvisiere (Schilder), sowie um kombinierte Vorrichtungen, welche Augenschutz mit dem Schutz der Atemwege verbinden. Diese Vorrichtungen haben folgende gemeinsame Eigenschaften. Alle Schutzbrillen, Masken und Visiere werden am Kopf mittels spezieller Vorrichtungen fixiert. Dabei handelt es sich um Brillenbügel, Gummizüge, Bänder, spezielles Kopfgeschirr, klebrige Streifen usw. Jedes Produkt ist aus mehreren Teilen gefertigt und verfügt über eine komplexe Herstellungskonstruktion. Bei der Herstellung der genannten Vorrichtungen werden viele Materialien und viel Energie verwendet. Dies ist aus ökologischer Sicht nicht immer gerechtfertigt, da die Herstellung von Kunststoff mit einem hohen Materialverbrauch verbunden ist. Dies betrifft hauptsächlich Einwegprodukte. Diese Produkte sind nicht zur Sterilisation bestimmt. Dies bedeutet, dass ein Arzt, der mit infizierten Patienten arbeitet, einen Monat lang viele Einwegbrillen verwendet. Dies schafft organisatorische Probleme und erhöht die Kosten für medizinische Dienstleistungen. Daher sind in der Medizin Produkte, die mehrfach verwendet werden können, häufiger anzutreffen. Bereits bekannte Schutzvorrichtungen für Augen, welche für mehrfache Anwendungen gedacht sind, müssen nach jeder Benutzung desinfiziert bzw. sterilisiert werden. Dies benötigt die Verwendung von Desinfektionslösungen und Reinigungstüchern. Dies erhöht die mit ihrer Verwendung verbundenen Kosten. Alle oben genannten Geräte benötigen spezielle Verpackungen, da sie während der Lagerung leicht beschädigt oder kontaminiert werden können. Die Produktion, Lagerung und Aufbereitung dieser Vorrichtungen ist deutlich aufwendiger als bei dem vorgeschlagenen Produkt.

Näher an die angebotene Schutzvorrichtung kommen die transparenten Kunststoffschilder. Diese Vorrichtungen bestehen aus einer Kunststoffplatte und einem speziellen Element zur Befestigung am Kopf. Diese Vorrichtungen schützen die Augen vor dem Eindringen von Tröpfchen, Spritzer und harten Teilchen während der Arbeit. Die Schutzschilder bringen mehr Komfort als eine Brille. Diese Vorrichtungen haben auch Nachteile. Kunststoffplatten sind in großem Abstand von den Augen befestigt. Da die Luft zwischen Plastikschild und Gesicht frei zirkuliert, können die sich in der Luft befindenden Viren und Bakterien in die Augen gelangen. Daher sollte medizinisches Personal, das mit infektiösen Patienten arbeitet, auch eine Schutzbrille tragen. Alle oben beschriebenen Vorrichtungen sollten an besonderen Orten gelagert und desinfiziert werden. Es erfordert zusätzliche Betriebskosten. Sie bestehen aus robustem und dickem Kunststoff. Daher sind sie teurer als Einweg-Plastikbrillen.

Wenn es nur um Aerosole und Spritzwasser geht, sind dünne Einweg-Schilder viel angemessener als robuste Kunststoffschilder. Diese Produkte werden häufig in Friseursalons eingesetzt. Es stehen viele verschiedene Varianten zur Verfügung, z.B: "Microblading Disposable Makeup Shower Haircutting Face Shields"; "Permanent Makeup Shower Face Shields Visors", usw. Es handelt sich dabei um ein durchsichtiges Blatt aus PET Kunststoff, welches auf die Stirn des Klienten geklebt wird, bevor an dessen Haaren gearbeitet wird. Dies ist eine dünne Folie, die an den Rändern mit einer Schicht aus klebrigem Kunststoff beschichtet ist.

Es gibt auch eine andere Modifikation. Die Kunststofffolie wird an einen Reifen geklebt, der mit einer Schnur am Kopf des Kunden befestigt ist ("Eye Protector Face Line Film"). Diese Vorrichtungen schützen das Gesicht des Kunden während einer einmaligen Haarbehandlung. Danach werden sie weggeworfen. Die oben beschriebenen Einweg-Produkte basieren auf Patent US4856535A "Protective Face Shield". Es ist eine Vorrichtung zum Schutz des Gesichts von Kunden beim Waschen. Dies ist ein Schild aus transparentem Kunststoff, der an den Rändern mit einem Streifen aus selbstklebendem Kunststoff bedeckt ist. Die Vorrichtung wird gegen die Haut gedrückt und haftet daran. Da sich diese Art von Schutzschild vor der Nase des Menschen befindet, beschlägt es sich während der Atmung.

Es gibt auch Patente für ähnliche Geräte: Patent EP 0 990 432 A, Eine Gesichtsschutzvorrichtung mit selbstklebenden Streifen, die auf die Stirnhaut aufgebracht wird während einer Haarbehandlung. In der kanadischen Patentschrift CA 2,109,500A1 ist eine transparente Schutzvorrichtung offenbart, die den Gesichtsbereich gegen Haarschnitt, Haarspray schützt und dadurch gekennzeichnet ist, dass diese Vorrichtung mit Bändern oder Bügeln am Kopf befestigt wird.

Alle diese Einweg-Vorrichtungen haben die gleichen Nachteile wie wiederverwendbare Schutzvisiere. Sie schützen die Augen ausschließlich nur vor Wasser und Spritzern. Kontaminierte Luft zirkuliert frei zwischen Plastik und Augen. Daher schützen sie die Augen nicht vor gefährlichen biologisch aktiven Mikropartikeln.

Alle oben beschriebenen Vorrichtungen sind nur für passive Dienstempfänger bestimmt. Diese Produkte sind nicht dazu gedacht, die Augen vor Aerosolen und kontaminiertem Bio-Staub zu schützen.

Eine weitere technisch naheliegende Lösung ist die Vorrichtung, wie im Patent US20040016037A1 beschrieben. Es handelt sich um Einweg-Schutzbrillen. Diese Vorrichtung wird aus einem Kunststoffblatt ausgeschnitten. Einweg-Schutzbrillen können nicht ohne Bänder oder Schnüre am Gesicht halten. Daher unterscheiden sie sich nicht von anderen Einweggläsern. Darüber hinaus sind sie im Vergleich zu der vorgeschlagenen Vorrichtung teurer in der Herstellung, sofern sie aus dickem Kunststoff bestehen.

Alle bekannten Schutzvorrichtungen, die dem aktuellen Stand der Technik entsprechen, haben oben beschriebene Nachteile.

Die Erfindung basiert auf der Aufgabe, eine günstige, einfache und umweltfreundliche Augenschutzvorrichtung bereitzustellen, die einerseits einen extrem hohen Schutz und andererseits einen sehr guten Tragekomfort bietet. Diese Aufgabe wird erfindungsgemäß durch die in dem Anspruch 1 angegebenen Merkmale gelöst. Die vorgeschlagene Vorrichtung erfüllt erfolgreich die Funktionen aller bekannten Schutzvorrichtungen. Die Vorrichtung vereint die Funktion einer Schutzbrille mit der eines Schutzschildes und bietet einen guten Schutz gegen gefährliche Mikropartikel. Sie ist kompakt. Bequem zu verstauen und einfach zu bedienen. Für diese Schutzvorrichtung sind keine Befestigungselemente erforderlich. Diese Vorrichtung ist sehr leicht. Die Verwendung ist viel komfortabler als die Verwendung bekannter Masken und Brillen. Darüber hinaus ist die vorgeschlagene Vorrichtung viel billiger und einfacher herzustellen als jede andere Schutzvorrichtung, die Herstellung verbraucht weniger Energie und Material.

Der Kern der Erfindung liegt darin, dass diese Aufgaben mit einer Kunststofffolie mit elektrostatischen Eigenschaften gelöst werden. Es gibt viele Materialien, die durch Reibung ihre elektrische Neutralität verlieren, so dass sich statische Aufladung ansammelt. Die Wirkung dieser Kraft (Kohäsionskraft, Van der Waalssche Kraft) nimmt zu, wenn sich Objekte einander nähern. Dank dieser Kraft beginnen die Objekte, sich zusammenzuziehen. Die Wirkung dieser Kraft kann in der Natur beobachtet werden. Elektrostatische, Van der Waalssche Kraft ermöglicht es beispielsweise Geckos, Spinnen und anderen kleinen Insekten, auf Wand, Fenster oder Decke zu kriechen (https://www.chemie.de/lexikon/Van-der-Waals-Kräfte.html; https://www.ingenieur.de/technik/fachbereiche/nanotechnologie/ geckos-haften-elektrostatikfuer/). Es gibt viele Theorien, die dies auf verschiedene Weise erklären. Sie alle erklären jedoch das gleiche physikalische Phänomen.

Die bekannten elektrostatischen Eigenschaften können in der Medizin angewendet werden. Es ist bekannt, dass dünne Plastikfolien, wenn Sie auf den menschlichen Körper, Glas, Plastik oder Metall aufgetragen und gerieben werden, fest an der Oberfläche haften. Besonders stark elektrostatisch aufladbare Materialien sind: Styropor, Polyacrylnitril (PAN), Polyethylen (PE), Polypropylen (PP), Polytetrafluorethylen (PT-FE), Polyvenylchlorid (PVC), Cellulosehydrat (Zellglas, Zellophan, Cellophan), Acetat, Celluloid, Polyester. Diese Eigenschaften der oben beschriebenen Folien können verwendet werden, um spezielle Augenschutzmasken herzustellen, die nach bionischen Prinzipien arbeiten.

Der Zweck der vorliegenden Erfindung ist es, eine Vorrichtung zu entwickeln, die die Prinzipien der Elektrostatik verwendet. Es geht um eine Gesichtsmaske, die mit Kohäsionskräften auf der Haut befestigt wird. Hierin wird die Augenschutzmaske entwickelt, die auf elektrostatisch geladenem Film basiert. Diese Vorrichtung benötigt keine zusätzlichen Teile oder Klebestreifen, um sie auf dem Gesicht zu befestigen. Darüber hinaus verfügt diese Vorrichtung über üblich physikalische Funktionen gewöhnlicher Brillen und Masken. Sie schützt die Augen und Schleimhäute vor Farbtropfen, Desinfektionsmitteln, Schmutz, feinen Spänen bei der Arbeit mit Holz usw. Die kleinen schädlichen Partikel (z.B. Staub, Aerosole, Bakterien usw.) haften an der Oberfläche der elektrostatisch geladenen Maske und gelangen nicht in die Augen.

Die flexible Maske aus einem transparenten, statisch geladenen Film hat große Vorteile gegenüber bekannten Schutzbrillen und Schutzschirmen. Der Vorteil der Maske besteht darin, dass sie aus einer homogenen Kunststofffolie besteht und keine Befestigungselemente benötigt.

Die Vorrichtung haftet gut auf der Haut. Die elektrostatische Aktivierung der Vorrichtung erfolgt durch Reiben der Oberfläche der Maske mit der Hand. Um die Haftung zu verbessern, wird die Haut vor dem Auftragen der Maske befeuchtet. Nach der Aktivierung haftet die Vorrichtung von selbst auf der Haut. Diese flexible Maske passt gut ins Gesicht und schützt zuverlässig die Augen. Gleichzeitig verliert sie bei längerem Gebrauch nicht ihre Schutzeigenschaften.

Diese Vorrichtung ist für den einmaligen Gebrauch bestimmt. Daher muss sie nach ihrer Anwendung nicht sterilisiert und gereinigt werden. Die Produktionskosten für diese Vorrichtung sind um ein Vielfaches günstiger als die Kosten für herkömmliche Einweg- und Mehrwegmasken und Brillen.

Die Vorrichtung kann zusammen mit einer Atemschutzmaske verwendet werden.

Die Vorrichtung wird aus Materialien hergestellt, welche unbedenklich für die Gesundheit des Menschen sind.

Sie benötigt keine bestimmten Umstände und Aufwände bei Lagerung und Wartung.

Sie ist platzsparend, verliert ihre Eigenschaften nicht bei langfristiger Aufbewahrung. Ein Set, bestehend aus mehreren einzelnen Folien-Augenschutzmasken, lässt sich problemlos in der Brusttasche eines Hemdes oder einer Kitteltasche verstauen.

Die Vorrichtung ist einfach und bequem in ihrer Anwendung, sie weist keine Nachteile auf, die bekannten Schutzbrillen und - masken inhärent sind.

Die Vorrichtung kann ebenfalls zum Augenschutz bei Kindern verwendet werden.

Die Anwendung der Vorrichtung stört nicht beim Tragen einer üblichen Brille bzw. einer Dioptrien- oder Lupenbrille.

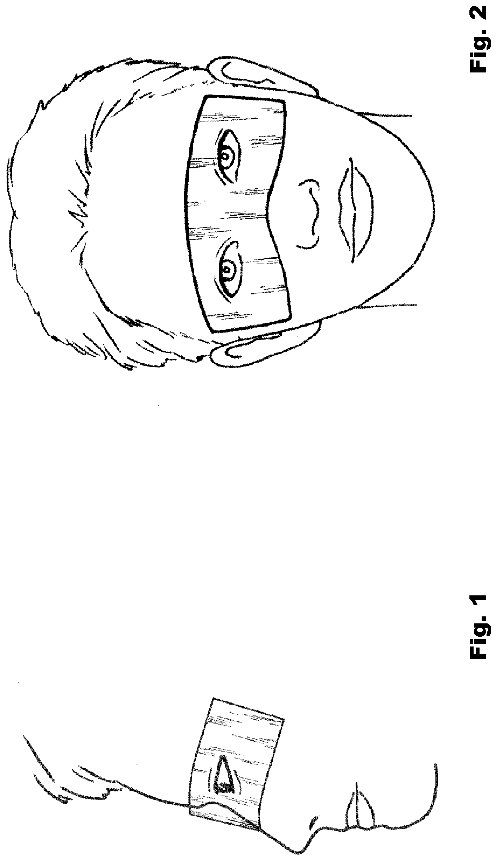

Die Figuren 1 und 2 zeigen ein bevorzugtes Ausführungsbeispiel er erfindungsgemäßen Maske bzw. die Verwendung eines Kunststofffilms als Maske - im Regelfall bevorzugt immer, ohne zusätzliches Halteband oder Haltevorrichtung
Die Erfindung hat die folgenden Merkmale. Die Vorrichtung ist dadurch gekennzeichnet, dass sie aus transparentem, flexiblem, elektrostatisch aktivierbarem, 0,025 - 0,5 mm dickem, durchsichtigem Film auf Basis von Polyacrylnitril (PAN), Polyethylen (PE), Polypropylen (PP), Polytetrafluorethylen (PTFE), Polyvenylchlorid (PVC), Cellulosehydrat (Zellophan, Cellophan), thermoplastischem Polyester (PET) besteht. Man kann als Faustformel sagen, dass Material verwendet wird, dessen Tendenz zu elektrostatischen Aufladung so groß ist, dass es sich durch Reiben mit der zumeist bloßen Hand derart aktivieren lässt, dass es auf der zumeist bloßen Gesichtshaut des Benutzers haftet und nicht unter dem Einfluß seines Eigengewichts herabfällt.

Die Augenschutz-Maske 1 wird aus einem transparenten Film herausgeschnitten, aus dem ein Rechteck von 21-23 cm Breite B und 6-10 cm Länge L gebildet wird. Typischerweise wird dieser Film so verwendet und dann an das Gesicht angesetzt, dass seine Breite B in etwa parallel zu der durch der durch die Augen des Trägers definierten Horizontallinienschar verläuft. In der Mitte des Rechtecks befindet sich ein vertikaler Schnitt, der mindestens die Hälfte der Länge des Rechtecks erfasst, oder ein halber Ellipsenausschnitt, oder vertikale Falte, die zwei Teile des Blattes trennt. (In dieser Ausführungsform wird das Blatt gefaltet. Es wird vor Gebrauch geöffnet).
Die Vorrichtung wird so aufgetragen, dass sich die Nase zwischen den eingeschnittenen Teilen (oder in der Falte) des Films befindet. Um die Haftung der Vorrichtung zu verbessern, wird die Haut in Stirn und Schläfen optional mit Feuchtigkeit versorgt. Die Vorrichtung wird fest auf die Haut gedrückt und 3-4 Mal mit den Händen geglättet. Danach haftet die Vorrichtung an der Haut und hält sich selbständig. Bänder, Schnüre, Kleber oder andere spezielle Geräte zur Befestigung der Vorrichtung am Gesicht sind nicht erforderlich.

Im Regelfall, bevorzugt immer, ist der Film so dick, dass er zwar flexibel aber in sich im Wesentlichen formstabil ist. Von einer Folie wird er sich dann dadurch unterscheiden dass er auch unter dem Einfluss eines Luftzugs, zumindest eines solchen, wie er in Gebäuden auftritt, bevorzugt generell, nicht die Tendenz hat Flatterbewegungen auszuführen. In vielen Fällen ist der Film so dick, dass er sich, anders als eine Folie, nicht reversibel knüllen und wieder glattziehen lässt. Die bevorzugte Materialstärke liegt bei 0,1 mm und mehr, idealerweise bei 0,25 mm und mehr

Im Regelfall, bevorzugt immer, sind die Materialstärke des Films und seine Tendenz zu elektrostatischen Haftung so aufeinander abgestimmt, dass der Film als ein über das Gesicht des Trägers verlaufender Bogen verwendet werden kann und auch in den Bereichen zwischen Ohr und benachbartem Auge anliegt und die elektrostatischen Anziehungskräfte groß genug sind um zu verhindern, dass sich der Film unter dem Einfluss seiner elastischen Rückstellkräfte wieder in seine ungekrümmte Ausgangsform zurückbewegt

Die Vorrichtung blockiert das Eindringen von Mikropartikeln in die Augen infolge sowohl mechanischer als auch elektrostatischer Fixierung. Mikropartikel mit der gleichen Ladung wie die Folie werden daraus elektrostatisch entfernt. Mikropartikel mit entgegengesetzter Ladung haften an der Außenfläche der Maske und gelangen daher nicht in die Augen.

Da die Vorrichtung die Augen vollständig bedeckt, sind ihre mechanischen Schutzeigenschaften höher als die von Einwegbrillen und Schilder.

Die Vorrichtung kann an jede Gesichtsgröße angepasst werden. Die Vorrichtung kann an den Rändern abgeschnitten werden. Die Größe kann mit einer Schere verringert werden.

Die Vorrichtung erfordert keine Pflege. Nach Gebrauch wird sie entsorgt.

Bei der Herstellung werden Kunststoffe verwendet, die für die Lebensmittelindustrie und die Medizin zugelassen sind.

Die vorgeschlagene Vorrichtung kann gleichzeitig mit Atemschutzmasken verwendet werden.

Da diese Einwegmaske sehr kompakt und einfach zu bedienen ist, kann sie in die Standardausrüstung von Ärzten, Pflegern, Polizisten und Militärpersonal aufgenommen werden.

## Patentansprüche

1. Vorrichtung zum Schutz der Augen vor biologisch gefährlichen Mikropartikeln aus transparentem, flexiblem, elektrostatisch aktivierbarem Film, der nach dem Reiben mit den Händen das Gesicht bedeckt und ohne Befestigungselemente an der Haut haftet.

2. Vorrichtung nach Anspruch 1, **dadurch gekennzeichnet, dass** die Vorrichtung aus einem 0,025 - 0,5 mm dicken durchsichtigen Film auf Basis von Polyacrylnitril (PAN), Polyethylen (PE), Polypropylen (PP), Polytetrafluorethylen (PTFE), Polyvenylchlorid (PVC), Cellulosehydrat (Zellophan, Cellophan), thermoplastischem Polyester (PET) besteht.

3. Vorrichtung nach Anspruch 2, **dadurch gekennzeichnet, dass** die Vorrichtung aus einem Blatt herausgeschnitten wird, aus dem ein Rechteck von 21-23 cm Breite und 6-10 cm Länge gebildet wird. In der Mitte des Rechtecks befindet sich ein vertikaler Schnitt, der mindestens die Hälfte der Länge des Rechtecks erfasst, oder ein halber Ellipsenausschnitt, oder vertikale Falte, die zwei Teile des Blattes trennt.

4. Vorrichtung nach Anspruch 3, **dadurch gekennzeichnet, dass** die Vorrichtung so aufgetragen wird, dass sich die Nase zwischen den eingeschnittenen Teilen (oder in der Falte) des Films befindet. Die Vorrichtung wird fest auf die Haut gedrückt und 3-4 Mal mit den Händen geglättet. Danach haftet die Vorrichtung an der Haut und hält sich selbständig. Um die Haftung der Vorrichtung zu verbessern, wird die Haut in Stirn und Schläfen mit Feuchtigkeit versorgt.

5. Vorrichtung nach Anspruch 4, **dadurch gekennzeichnet, dass** die Vorrichtung das Eindringen von Mikropartikeln in die Augen infolge sowohl mechanischer als auch elektrostatischer Fixierung blockiert.

6. Vorrichtung nach Anspruch 5, **dadurch gekennzeichnet, dass** die Vorrichtung an jede Gesichtsgröße angepasst werden kann. Die Vorrichtung kann an den Rändern mit einer Schere abgeschnitten werden.

7. Verwendung einer Vorrichtung nach einem der vorhergehenden Ansprüche zum Schutz der Augen vor biologisch gefährlichen Mikropartikeln aus transparentem, flexiblem, elektrostatisch aktivierbarem Film, der nach dem Reiben mit den Händen das Gesicht bedeckt und ohne Befestigungselemente an der Haut haftet.
